# EUROPEAN PATENT SPECIFICATION

(11) **EP 3 386 957 B1**
(45) Date of publication and mention of the grant of the patent: **30.09.2020**
(21) Application number: 16843223.5
(22) Date of filing: 12.12.2016
(51) Int. Cl.: C07D 305/14

(54) **PROCESS FOR THE PURIFICATION OF 10-DEACETYL BACCATIN III**
VERFAHREN ZUR REINIGUNG VON 10-DEACETYL BACCATIN III
PROCÉDÉ DE PURIFICATION DE 10-DEACETYL BACCATIN III

(30) Priority: 11.12.2015 HU 1500603
(43) Date of publication of application: 17.10.2018
(73) Proprietor: RotaChrom Technológiai és Szolgálató Zártkörüen Müködö Részvénytársaság, 6000 Kecsekemét (HU)
(72) Inventor: LORÁNTFY, László, 2370 Dabas (HU); NÉMETH, László, 2370 Dabas (HU)
(74) Representative: Szentpéteri, Zsolt
(86) International application number: PCT/HU2016/050061
(87) International publication number: WO 2017/098291

(56) References cited:
- FR-A1- 2 903 104
- US-A- 4 814 470
- US-A- 6 008 385
- FOUCAULT A P ET AL: "Counter-current chromatography: instrumentation, solvent selection and some recent applications to natural product purification", JOURNAL OF CHROMATOGRAPHY A, ELSEVIER, AMSTERDAM, NL, vol. 808, no. 1-2, 29 May 1998 (1998-05-29), pages 3-22, XP004122655, ISSN: 0021-9673, DOI: 10.1016/S0021-9673(98)00121-6

## Description

### The background of the invention

The subject of the invention relates to a method for separating 10-deacetylbaccatin III and to the 10-deacetylbaccatin III solution produced in this way.

### The state of the art

Several anticancer chemotherapy drugs have been developed from taxane compounds belonging to the diterpene class, widely used examples of which include docetaxel, cabazitaxel and paclitaxel, among which docetaxel and paclitaxel are included in the list of medicines deemed to be the most important by the WHO (WHO Model List of Essential Medicines 2016).

Their industrial production may take place using a natural method, or using a semi-synthetic or synthetic method. Paclitaxel may also be found in nature, in very small amounts however, therefore it is usually produced from natural precursors during synthesis. Docetaxel and cabazitaxel are compounds synthetically produced from natural taxol precursor. The total synthesis of these compounds is known (Danishefsky, 1996) as is their production using biotechnology techniques (Navia-Osorio, 2002), which recently have started to gain an industrial role.

The precursor of the compounds mentioned is the compound called formula 1 10-deacetylbaccatin III (hereinafter: 10-DAB III):

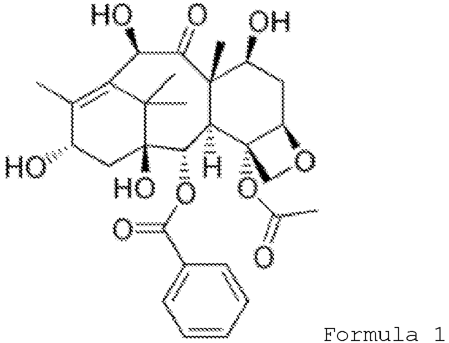

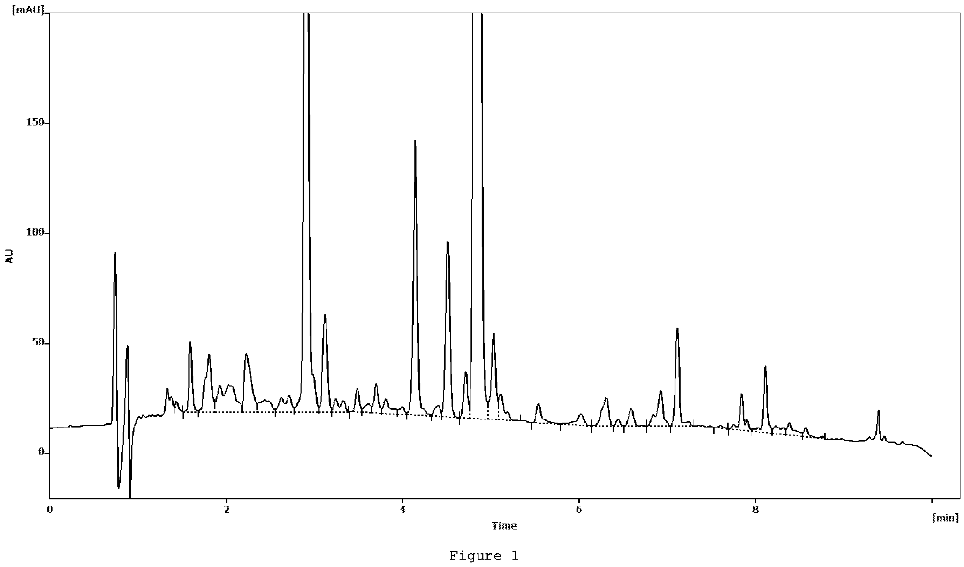

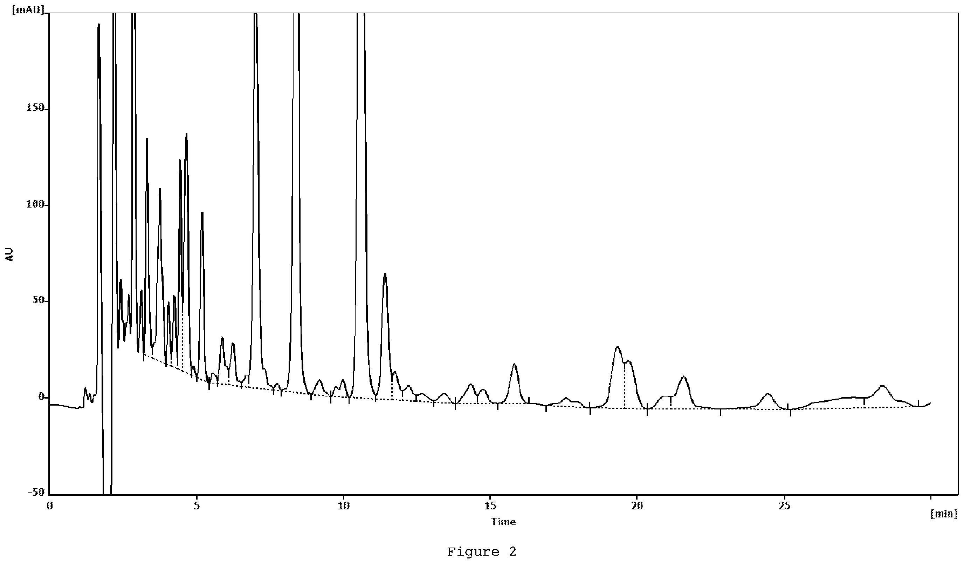

10-DAB III can be found in and extracted from several species of yew tree (such as the Taxus Baccata and Taxus Wallichiana species - (Németh-Kiss, 1996)), however the substance obtained in this way contains too much contaminant. Many parts of the plant (leaves, branches, bark) contain these substances, in varying amounts, the most economic production method being extraction from the leaves. However, a purer substance than this is required for use as a precursor in the pharmaceutical industry. The purity of the initial materials is characterised by the chromatograms shown in figures 1 and 2 (own measurements, Taxus Baccata (figure 1) and Taxus Wallichiana (figure 2) extracts).

Several patent applications deal with the separation of the 10-DAB III compound from its contaminants (such as patent applications US5969165A and US6002025A). As a consequence of the above the primary purpose of the purification of mixtures containing 10-DAB III is for the product of appropriate purity from the point of view of the pharmaceutical industry to be suitable for the synthetic or biosynthetic production of docataxel or paclitaxel. The expected purity depends on the synthesis method of the taxols, however purity above 98% or 99% is a general expectation.

Several experiments have been performed relating to the synthetic production of the 10-DAB III compound (Danishefsky, 1996).

It may be seen that the 10-DAB III solution serving as a pharmaceutical industry precursor may be produced in several ways, however, it can be said of the solutions obtained in this way that their purity is insufficient.

Preparative high performance liquid chromatography (prep-HPLC) methods are known of for the purification of 10-DAB III solution (see for example patent application number US5670673A). These procedures are characterised by high performance, and 10-DAB III solution with purity of >98% can be produced with them with a yield of >60%. However, these methods typically involve great use of solvent and adsorbent, which makes the production of 10-DAB III for use in the pharmaceutical industry costly.

Centrifugal Partition Chromatography (abbreviated as: CPC) is a chromatographic technique that is both very efficient and cost-effective. Several patent applications deal with the CPC purification of contaminated 10-DAB III mixture (see USA patent number 6,008,385 for example; and USA patent number 5,723,635). The methods disclosed in these patent documents focus on the purification of the extract from certain contaminants, by using them it is not possible to universally remove all contaminants, so it is not possible to obtain a 10-DAB III product with a purity of >99% using these methods (Margraff, 1995). It is a general problem in the case of natural extracts that the composition of the extracts varies (depending on season, species and source). Consequentially the composition of the aforementioned contaminants varies as well, therefore the method used for purification must be robust for these changes, which requirement is not met by the solutions disclosed in the above patents.

Also, the solutions according to these patents cannot be upscaled, and so they are not suitable for industrial scale purification for the following reasons. During the use of the heptane - ethyl acetate - methanol - water (1:2:1:2) system disclosed in USA patent number 6,008,385, the ethyl acetate decomposes (it hydrolyses with water and esterifies with methanol), therefore the composition of the solvent system, which is one of the most important parameters of CPC separation, changes. Another problem is posed by that in the case of the use of a four-component system, setting the recycled solvent to its original composition demands an expensive and costly instrument background (gas chromatography testing, Karl Fischer titration). In the case of the other solvent system disclosed in the patent, which uses a mixture of methyl isobutyl ketone (MIBK), acetone and water, both phases have a high boiling point. As the 10-DAB III can be obtained from the purified fraction by evaporation, and 10-DAB III is a heat-sensitive material, it is not preferred to exert it to temperatures above 40 °C, or other heat loads for extended periods. In the case of a solvent with a high boiling point a great vacuum needs to be used, however, vacuums higher than 100-150 mbar are costly to use during industrial production. Another problem in the case of the method disclosed in the patent is that as 10-DAB III only dissolves in a large amount in the solvent system used when heated, the rotation of the centrifuge must be slowed down before every sample injection cycle, a separate pump must be used for injecting the sample, then the centrifuge must be brought back up to speed, otherwise there is a risk of blockages occurring due to precipitating sample. Such a process demands a great deal of energy and time due to the great inertia of industrial centrifuges, and during an industrial process the risk of blockage is impermissible.

Furthermore, the inventors of the present invention were also unable to produce 10-DAB III with a purity of >99% using the methods disclosed in the patent documents referred to. The reason for this is that although the system consisting of a mixture of methyl isobutyl ketone, acetone and water has excellent selectivity for the contaminant 19-OH-10-DAB III, the selectivity given for other contaminants is very low. Therefore if the amount of the latter contaminants is large, their separation cannot be realised without significant loss of yield.

### Brief description of the invention

In accordance with the above, the objective of the invention is to provide a CPC method that overcomes the above problems of the state of the art, i.e. the method is not contaminant-specific, instead it can be universally used for cleaning solvents containing various contaminants and with which mixtures containing 10-DAB III can be effectively purified with greater purity as compared to the solutions to date that satisfies pharmaceutical industry demands, in addition to which the method may be easily used on the industrial scale. We set our objective to be the elaboration of such a method. The objective of the invention is also to provide 10-DAB III solution of appropriate purity.

Our invention is based on the surprising recognition that if solvent mixtures of MEK/water, MTBE/acetone/water and/or DCM/MeOH/water are used when purifying a mixture containing 10-DAB III using centrifugal partition chromatography (CPC) then the above objective can be achieved.

In accordance with the invention the task was solved with the implementation of the CPC method according to claim 1.

Individual preferable embodiments of the invention are determined in the dependent claims.

The invention relates to a centrifugal partition chromatography (CPC) method serving for the separation of 10-deacetylbaccatin III, where the solution to be purified containing 10-deacetylbaccatin III is purified in at least one CPC step, and where during the CPC step one of the following is used as the solvent mixture: MEK/water, MTBE/acetone/water, and DCM/MeOH/water.

A preferable embodiment of the invention relates to a centrifugal partition chromatography (CPC) method serving for the separation of 10-deacetylbaccatin III, where the solution to be purified containing 10-deacetylbaccatin III is purified in at least two CPC steps, and where during the individual CPC steps one of the following is used as the solvent mixture in such a way that during the method the at least two CPC steps are carried out with different solvent mixtures: MEK/water, MTBE/acetone/water, and DCM/MeOH/water.

According to a preferable embodiment of the invention the solution containing 10-DAB III is purified in at least three CPC steps in such a way that during the method the at least three CPC steps are performed using different solvent mixtures.

According to another preferable embodiment of the invention during a purification procedure where at least two CPC steps are implemented, the CPC steps are performed one after the other without the interposition of other purification steps.

According to yet another different preferable embodiment of the invention where at least two CPC steps are implemented, the CPC step using the DCM/MeOH/water solvent mixture is performed not as the last CPC step.

According to another preferable embodiment of the invention the individual solvent mixtures contain the solvents in the following volume ratios:
MEK/water: 1:2;
MTBE/acetone/water: 4:4:3;
DCM/MeOH/water: 3:4:3;
where the individual volume ratios deviate from the above values by a maximum of 20%, preferably by a maximum of 15%, more preferably by a maximum of 10% and most preferably by a maximum of 5%.

According to another preferable embodiment of the invention pre-purification is implemented for removing the chlorophyll content. The pre-purification is performed using a CPC step or a liquid chromatography step.

According to another preferable embodiment of the invention the solution to be purified containing 10-deacetylbaccatin III is a plant extract, preferably an extract prepared from any part of a yew tree species, even more preferably an extract made from mainly the leaves of a yew tree species. The yew tree species is preferably selected from the Taxus Baccata or Taxus Wallichiana species.

In the figures
Figure 1 shows the HPLC-UV chromatogram of the Taxus Baccata extract;
Figure 2 shows the HPLC-UV chromatogram of the Taxus Wallichiana extract;
Figure 3 shows the chromatogram of the product of the purification performed in the MEK/water biphasic solvent system;
Figure 4 shows the purity of the 10-DAB III obtained during the pre-purification carried out in the DCM/MeOH/water 3:4:3 volume ratio mixture due to the presence of chlorophyll;
Figure 5 shows the chromatogram of a sample purified in the MTBE/acetone/water solvent mixture following purification performed using the solvent mixture of DCM/MeOH/water.

In the present specification the following abbreviations are used for the individual solvents:
DCM: dichloromethane
MEK: methyl-ethyl-ketone
MeOH: methanol
MIBK: methyl-isobutyl-ketone
MTBE: methyl tert-butyl ether

### Detailed description of the invention

Throughout the present patent specification the concepts "purification" and "separation" are understood as having the same meaning, unless another meaning may be concluded from the given context, namely the purification of solutions containing 10-DAB III to remove contaminants with a result that provides a 10-DAB III solution with the purity according to the objectives.

Throughout of the present invention a CPC procedure means methods according to the invention containing at least one CPC step, preferably at least two CPC steps, even more preferably at least three CPC procedurial steps. The expression CPC procedure throughout the present invention does not exclude that other non-CPC purification steps are implemented during the method. The individual CPC steps are understood as meaning a complete CPC purification cycle performed with a given solvent mixture.

In accordance with the present invention solutions containing 10-DAB III can be separated from contaminants with great efficiency by selecting one, two or all three of the three solvent mixtures (MEK/water, MTBE/acetone/water, and DCM/MeOH/water) for CPC steps. This means that a solution to be purified containing 10-DAB III is subjected to a first CPC procedurial step using a solvent mixture selected from among those above, then, optionally, the partially purified 10-DAB III solution obtained in this way is subjected to a second CPC procedurial step, if necessary, using another solvent mixture selected from among those above that is different to that used in the first CPC procedurial step. As is obvious for a person skilled in the art, the said first and said second CPC procedurial steps can be preceded by, interposed with or followed by other CPC or other (e.g. HPLC) procedurial steps.

According to the invention then the solvent mixtures according to the following Table 1 may be used in the said first and said second CPC procedurial steps during the implementation of the CPC procedure according to the invention:

**Table 1: possible solvent mixtures during the implementation of the CPC procedure**

| first CPC procedurial step | second CPC procedurial step |
|---|---|
| MEK/water | MTBE/acetone/water |
| MEK/water | DCM/MeOH/water |
| MTBE/acetone/water | MEK/water |
| MTBE/acetone/water | DCM/MeOH/water |
| DCM/MeOH/water | MEK/water |
| DCM/MeOH/water | MTBE/acetone/water |

Nevertheless, according to our experience very similar results are obtained from two different sequences of a solvent mixture pair, i.e. in the case of the implementation of two CPC procedurial steps it is substantially unimportant which is used first and which is used second.

In accordance with the present invention solutions containing 10-DAB III can be separated from contaminants with great efficiency by using preferably three solvent mixtures (MEK/water, MTBE/acetone/water, and DCM/MeOH/water) for three CPC procedurial steps. This means that a solution to be purified containing 10-DAB III is subjected to a first CPC procedurial step using a solvent mixture selected from among those above, then the partially purified 10-DAB III solution obtained in this way is subjected to a second CPC procedurial step using another solvent mixture selected from among those above that is different to that used in the first CPC procedurial step, then the partially purified 10-DAB III solution obtained in this way is subjected to a third procedurial CPC step using the solvent mixture remaining from the above, i.e. different to those used in the first and second CPC procedurial steps. As is obvious for a person skilled in the art, the said first, the said second and said third CPC procedurial steps may be preceded by, interposed with or followed by other CPC or other (e.g. HPLC) procedurial steps.

According to the invention then the solvent mixtures according to Table 2 may be used in the said first, said second and said third CPC procedurial steps during the implementation of the CPC method according to the invention:

**Table 2: possible solvent mixtures during the preferable implementation of the CPC method**

| first CPC procedurial step | second CPC procedurial step | Third CPC procedurial step |
|---|---|---|
| MEK/water | MTBE/acetone/water | DCM/MeOH/water |
| MEK/water | DCM/MeOH/water | MTBE/acetone/water |
| MTBE/acetone/water | DCM/MeOH/water | MEK/water |
| MTBE/acetone/water | MEK/water | DCM/MeOH/water |
| DCM/MeOH/water | MTBE/acetone/water | MEK/water |
| DCM/MeOH/water | MEK/water | MTBE/acetone/water |

Nevertheless, according to our experience very similar results are obtained from three different sequences of three solvent mixtures, i.e. in the case of the implementation of three CPC procedurial steps it is substantially unimportant which is used first, which is used second and which is used third.

Whether one, two or three CPC procedurial steps are required to purify a given contaminated 10-DAB III solution is determined by the contaminant profile of the solution to be purified, an example of which is shown in Table 3. It is conceivable that it is sufficient to use just one solvent mixture (for example, only MEK/water, or only MTBE/acetone/water or only DCM/MeOH/water). It may be necessary to implement two CPC procedurial steps due to the composition of the 10-DAB III solution to be purified, using carefully selected solvent mixtures, but it is possible that the implementation of three CPC procedurial steps is necessary.

The method according to the present invention is preferably implemented so that the said CPC procedurial step is performed with the said solvent mixtures, or in the case of the necessity for several CPC procedurial steps the steps are performed sequentially without interposing other purification steps. According to our experience, however, by using only the said one, two or three solvent mixtures according to the invention in CPC procedurial steps the majority of contaminated 10-DAB-III solutions may be purified to the desired quality. It is obvious for a person skilled in the art that between/after the individual CPC procedurial steps it is preferable to use evaporation.

According to a preferable form of implementation of the method according to the invention, if at least two CPC procedurial steps are used, the procedurial step using the DCM/MeOH/water solvent mixture is performed not as the last CPC procedurial step. This is because DCM is a toxic solvent, therefore it is important for the 10-DAB III for pharmaceutical industry uses contains as little residual DCM as possible. In practice this may be ensured if the solvent mixture containing DCM is used in the earliest possible procedurial steps of the method, as in this case during the later procedurial steps at least a part of the residual DCM can still be removed. Therefore if the DCM/MeOH/water solvent mixture is used during the implementation of the method according to the invention containing two CPC procedurial steps as one of the solvent mixtures, it is preferable if the DCM/MeOH/water solvent mixture is used as the one solvent mixture for the first CPC procedurial step. If three CPC procedurial steps are used during the implementation of the method according to the invention, then it is preferable to use the DCM/MeOH/water solvent mixture for the first or second CPC procedurial step, or more preferably for the first CPC procedurial step.

We recognised that in the interest of achieving outstanding purification results it is preferable to use the given solvents in the individual solvent mixtures at a given volume ratio. Namely, the given solvents are preferably used in the individual solvent mixtures in the following volume ratios:
MEK/water: 1:2;
MTBE/acetone/water: 4:4:3;
DCM/MeOH/water: 3:4:3.
It is obvious for a person skilled in the art that a certain degree of deviation from the above values is conceivable so that the purity of the 10-DAB III solution does not substantially change. Therefore, according to the present invention the above volume ratios are met if the volume ratios used deviate from the above values by a maximum of 20%, preferably by a maximum of 15%, even more preferably by a maximum of 10% and most preferably by a maximum of 5%.

If the solution to be purified containing 10-DAB III contains more chlorophyll than is desirable, it is preferable to subject the sample to pre-purification. This is typical in the case if the solution to be purified is an extract of green plant parts, an extract of yew tree leaves, for example. The pre-purification may be performed using a liquid chromatography method known to a person skilled in the art (see for example disclosure document number US 5670673 A), or by extraction performed with CPC, during which a chlorinated hydrocarbon or a mixture of this with other solvents is used as the solvent. The chlorinated hydrocarbon is preferably dichloromethane. In the case of exaggerated chlorophyll content it is especially preferable to perform the pre-purification with CPC, using dichloromethane, methanol and water at the approximate volume ratios of 3:4:3 (i.e. 3±0.3:4±0.4:3±0.3) as the solvent mixture. In this case the composition of the lower phase is 90% dichloromethane and 10% methanol, while the composition of the upper phase at this time is 55% methanol, 35% water and 10% dichloromethane. If pre-purification for removing the chlorophyll content is used, then following this, depending on the amount of residual chlorophyll in the solution to be purified and of the other contaminants, it can be decided whether to use the DCM/MeOH/water solvent mixture in the way presented above in (further) CPC procedurial step(s). Therefore, in the case of significant chlorophyll content it is conceivable that the DCM/MeOH/water solvent mixture is used twice, or even more times, during the entire purification process. It should be noted that in accordance with the invention not only DCM is suitable for removing the chlorophyll content in the pre-purification step as, optionally, other chlorinated hydrocarbons are also suitable, also the pre-purification step does not necessarily have to be performed with CPC, the traditional liquid chromatography process may also be suitable for this purpose.

Although 10-DAB III may be produced semi-synthetically and synthetically (see above), according to the state of the art it may be most preferably produced by extraction from various yew tree species. The extracts are usually produced from the green plant parts, primarily from the leaves. The two yew tree species most frequently used for this purpose are Taxus Baccata and Taxus Wallichiana. Therefore, the invention relates to the purification of solutions produced from preferably these yew tree species, preferably from their leaves. It is obvious for a person skilled in the art that while collecting the plant parts to be subjected to extraction other plant parts apart from the leaves may also be included in the material to be processed, which increases the diversity of the contaminants and even their amount as well. This is why the non-selective separation method according to the present invention is important.

High purity 10-DAB III solution suitable for use in the pharmaceutical industry may be obtained with the method according to the present invention. Therefore the present invention also relates to the 10-DAB II solution obtained with the method according to the invention.

10-DAB III dissolves well in few solvents, however, previously it was found that (Margraff, 1995) it dissolves better in a mixture of solvents than in a pure solvent on the condition that the one solvent dissolves 10-DAB III well on its own (acetone in a case known in the prior art).

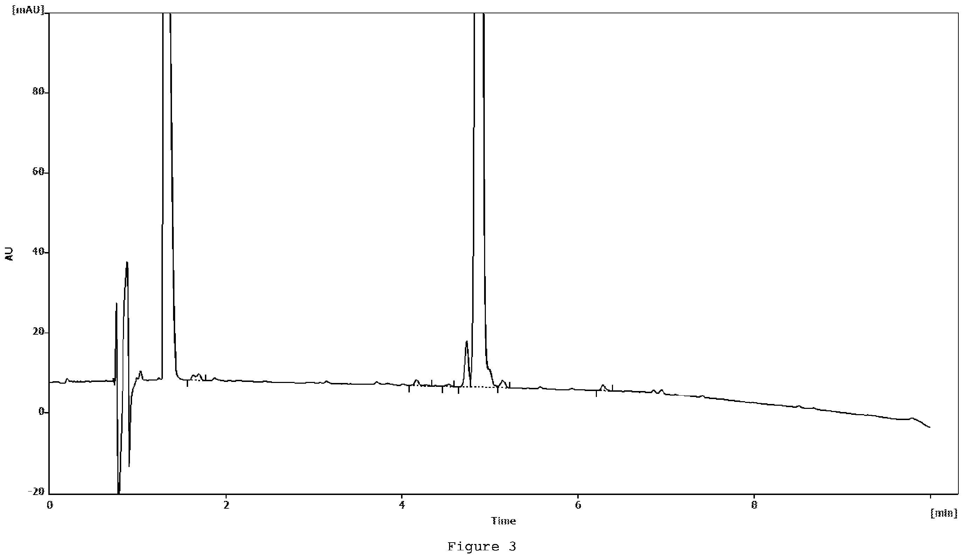

In the light of this it was surprising to experience that while 10-DAB III does not dissolve well separately in MEK or water, a biphasic system of the two (in which the upper phase is MEK saturated with water and the lower phase is water saturated with MEK) dissolves 10-DAB III, and the solubility in the upper phase of 80 mg/ml is way in excess of the expectations that could be presumed on the basis of the prior art. As such a degree of solubility may only be achieved in a mixture of MIBK, acetone and water after heating, after cooling and shaking the substance crystalized (Margraff, 1995). The partition coefficient of 10-DAB III is around 6. The method exhibits good selectivity on the basis of the later disclosed isocratic HPLC method for the RRT=0.95 and the RRT=0.84 contaminants. The chromatogram according to Figure 3 presents a product purified in such a solvent system.

It was also surprising to experience that the biphasic system formed by the mixture of methyl tert-butyl ether (hereinafter: MTBE), acetone and water is also suitable for dissolving 10-DAB III, if their volumetric mixing ratio is approximately 4:4:3, in which the upper phase is a mixture of 40% MTBE, 50% acetone and 10% water, and the lower phase is a mixture of 40% water, 50% acetone and 10% MTBE. 10-DAB III is insoluble separately in MTBE and in water, but it dissolves in both phases of the aforementioned ternary solvent mixture, solubility in the upper phase is 50 mg/ml. During use in chromatography this solvent system ensures shorter run times, as the partition coefficient of 10-DAB III is around 2, and has great selectivity for several contaminants, including contaminants with respect to which the MEK/water biphasic system is ineffective. Such contaminants on the basis of the HPLC method below are the RRT=0.68 and the RRT=0.87 contaminants. In the isocratic HPLC method both MEK/water and MTBE/acetone/water have great selectivity for the substance with the presumed structure 19-OH-10-deacetylbaccatin III with the relative retention time of RRT=0.48.

In samples in which there are a lot of contaminants present it is preferable to combine the CPC procedurial steps performed with the MEK/water and MTBE/acetone/water solvent mixtures due to the great degree of chromatographic orthogonality.

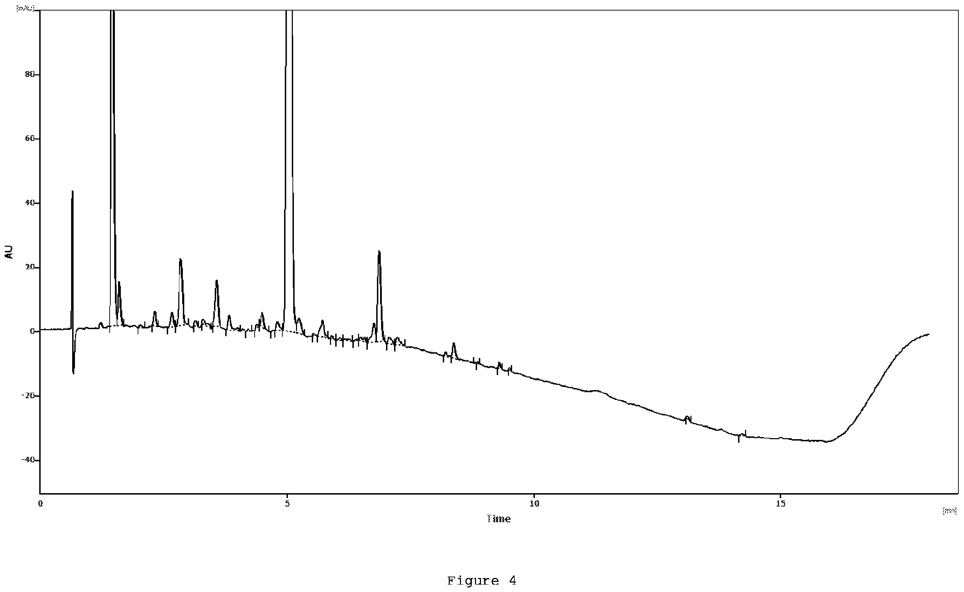

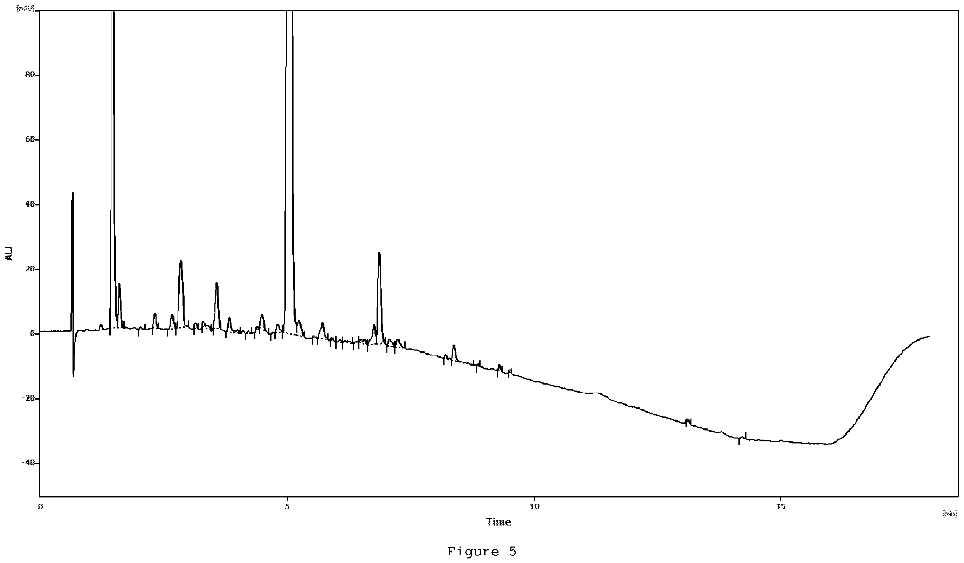

In samples where the removal of the chlorophyll has not yet taken place, during HPLC measurements the presence of peaks was experienced (for example RRT=1.05) for which the two CPC procedurial steps detailed above still had low selectivity, and due to this the yield was limited. Liquid chromatography or extraction performed with a chlorinated hydrocarbon is usually used for the separation of chlorophyll and related compounds. If this extraction is carried out with CPC, the efficiency and yield of the separation is significantly improved. A solvent system that may be used for this may be the dichloromethane, methanol and water mixture at an approximate volumetric ratio of 3:4:3. In this solvent system the partition coefficient of 10-DAB III is 0.95, while that of the aforementioned problematic contaminants is around 0.4-0.5. The chromatogram shown in Figure 4 shows the purity of the 10-DAB III pre-cleaned with this method. The chromatogram shown in Figure 5 shows a sample that was also purified in the MTBE/acetone/water system following purification in the previous DCM/MeOH/water system.

The HPLC methods used for the analysis:
1. Isocratic method
   - Column: Dr Maisch ReproSil-Pur C18-AQ (150x4.0 mm, 3 um)
   - Column thermostat: 35 °C
   - Flow rate: 0.75 ml/min
   - Eluent: 28% acetonitrile, 72% water, 0.1% acetic acid
   - Detection wavelength: 230 +/- 5 nm
   - Injected volume: 10 ul
2. Gradient method
   - Colum: Phenomenex Kinetex C18 (100x3.0 mm, 2.6 um)
   - Column thermostat: 35 °C
   - Flow rate: 0.7 ml/min
   - Eluent A: water + 0.1% acetic acid
      Eluent B: acetonitrile
      Gradient program: 0 min 20% B, 5 min: 30% B, 11 min 40% B, 13 min 40% B, 14 min 20% B, 18 min 20% B
   - Detection wavelength: 230 +/- 5 nm
   - Injected volume: 5 ul

The following Table 3 shows the selectivity matrix of the individual CPC methods according to the invention, where the individual contaminants have been identified with their relative retention times (RRT).

**Table 3: the selectivity matrix of the individual methods according to the invention**

| Contaminant | RRT=0.48 | RRT=0.68 | RRT=0.87 | RRT=1.05 | RRT=1.47 | RRT=1.67 |
|---|---|---|---|---|---|---|
| MEK/water | +++ | +++ | +++ | + | +++ | +++ |
| MTBE/ | +++ | + | + | + | +++ | +++ |
| acetone/ water | | | | | | |
| DCM/MeOH/ water | + | + | + | +++ | + | + |

| | | | | | | |
|---|---|---|---|---|---|---|
| Legend: + low selectivity, ++ medium selectivity, +++ high selectivity | | | | | | |

With the complementary use of the three CPC procedurial steps (MEK/water; MTBE/acetone/water; DCM/MeOH/water) the 10-DAB III can be separated with great efficiency from almost all contaminants. This means that a partially purified product may be obtained by injecting the contaminated substance into a CPC containing the one solvent mixture. If this product is then evaporated and then injected once again into a CPC containing a different solvent mixture, then a product may be obtained with purity above 99.5%, in which the amount of every contaminant that may be detected with the previous HPLC method drops to under 0.1%.

The advantage of these systems is that by using distillation the solvents used may be more easily recycled, as opposed to the MIBK used in the CPC method known according to the state of the art (Margraff, 1995) the boiling points of the solvents used in the method according to the invention are much lower (the boiling points of the solvents mentioned in the present specification are known to a person skilled in the art from the prior art). As 10-DAB III is a thermolabile substance, it is preferable not to subject it to temperatures over 40 °C or to other heat loads for an extended period of time. However, in the case of a high boiling point substance a great vacuum needs to be used so the material is not subjected to a heat load when recovering the product. As during industrial production the use of great vacuums is costly, it is preferable to use volatile solvents with a lower boiling point, which condition the solvents according to the invention meet in full. In the case of the system disclosed in Margraff's patents a temperature of >60 °C is needed in the case of the vacuums generally used in the industry.

In the case of the two-component MEK/water system, after distillation, due to the presence of the two phases there is no need to set the appropriate phase composition as in the case of the mixtures with three or four component used during the method according to the state of the art (see the Margraff patent document).

In the case of CPC methods it is important to maintain the composition of the solvent system used (as an important parameter influencing separation) at a constant value, due to this in the case of systems with four components costly GC measurement or Karl-Fischer titration is required for the testing of the recycled solvent mixture.

However, in the case of the MTBE/acetone/water solvent mixture, after distillation the solvent composition may be easily set using simple density measurement, as there is a clear and linear relationship between density and composition, due to this this step may be easily automated. According to our experience more than 95% of the solvent can be recycled in practice. As a result of this the industrial upscaling of the method according to the invention does not come up against difficulties and the method is economic.

In the following the invention is presented with the help of examples.

### Example 1:

500 ml of MEK and 1 litre of water were mixed and then the phases were separated. An Armen SCPC-250 device with a column volume of 250 ml was filled with upper phase, then 2 g of 60% initial purity 10-DAB III dissolved in 20 ml of upper phase was injected. Following this the lower phase was pumped at a flow rate of 10 ml/min, then after 90 minutes upper phase pumping was started once again. The retention time of the main substance at this time is about 90 minutes, and 1.1 g of 99.2% purity product was obtained, which was crystalized by evaporation, then the moisture was removed in a vacuum furnace.

The solvent use for the injected material was 500 ml/gram, and 900 ml/gram when calculated for the product, which, however, could almost entirely be recycled.

### Example 2:

800 ml of MTBE, 600 ml of acetone and 600 ml of water were mixed, then the phases were separated. An Armen SCPC-250 device with a column volume of 250 ml was filled with upper phase, then 2.0 g of material dissolved in 40 ml of upper phase was injected. Following this the lower phase was pumped at a flow rate of 10 ml/min, then after 45 minutes upper phase pumping was started once again. The retention time of the main substance at this time is about 45 minutes, and 1.1 g of 99.1% purity product was obtained, which was crystalized by evaporation, then the moisture was removed in a vacuum furnace.

The solvent use for the injected material was 250 ml/gram, and 450 ml/gram when calculated for the product, which, however, could almost entirely be recycled.

### Example 3:

2 g of 60% initial purity 10-DAB III was purified similarly to Example 1, with the difference that the evaporated product was not crystalized. The 1.1 g of product obtained was injected once again according to that stated in Example 2, during which product with a purity of >99,5% was obtained, in the case of which the amount of every detectable contaminant (using the HPLC methods indicated above) was under 0.1%.

### Example 4:

3.0 g of 5% initial purity 10-DAB III active substance was injected into an Armen SCPC device with a column volume of 250 ml, which was filled with the lower phase of the DCM/MeOH/water (3:4:3) system, as stationary phase. 180 ml of upper phase, as mobile phase, was pumped at a flow rate of 10 ml/min, then the other components were forced out with 180 ml of stationary phase. Then during the collection of the fractions 50 ml of solution was obtained, which after evaporation resulted in 0.23 g of 60% purity 10-DAB III. This was purified further in the MTBE/acetone/water system on the basis of the method in Example 2, and 0.11 g of >99% purity 10-DAB III was obtained.

### Example 5:

A 100 g/l solution was prepared from a 60% purity sample of 10-DAB III. 20 1 of MEK and 40 l of water were mixed together, and the phases were separated. A RotaChrom rCPC device with a column volume of 2 l rotating at a speed of 600 rpm was filled with the upper phase of the former MEK/water solvent system. The flow rate was set to 300 ml/min, during which, after the pumping of the mobile phase, 30% of the stationary phase had been removed. 80 ml of sample was injected. The mobile phase was pumped for 40 minutes, and then the stationary phase was pumped for 6 minutes. During the purification 1 litre of product was collected, after the evaporation of which 4.0 g of 99% purity 10-DAB III was obtained.

The results of the method according to the invention may be summarised in the following:
- it may be easily upscaled;
- it has low organic solvent use; and
- it is able to produce 10-DAB III of high purity (>99%).

### References

Danishefsky, S. J. (1996). Total Synthesis of Baccatin III and Taxol. J. Am. Chem. Soc, 2843-2859.
Margraff, R. (1995). Preparative Centrifugal Partition Chromatography. In A. P. Foucault, Centrifugal Partition Chromatography (pp. 335-348). New York, Basel, Hong Kong: Marcel Deccer, Inc.
Navia-Osorio, A. (2002). Taxol ((R)) and baccatin III production in suspension cultures of Taxus Baccata and Taxus Wallichiana in an airlift bioreactor. Journal of Plant Physiology, 97-102.
Németh-Kiss, V. (1996). Taxol content of various Taxus species in Hungary. Journal of Pharmaceutical and Biomedical Analysis, 997-1001.

## Claims

1. Centrifugal partition chromatography (CPC) method serving for separating 10-deacetylbaccatin III, **characterised by** that a solution to be purified containing 10-deacetylbaccatin III is purified in at least one CPC procedurial step, and where during the CPC procedurial step one of the following is used as the solvent mixture: MEK/water, MTBE/acetone/water, and DCM/MeOH/water, where the individual solvent mixtures contain the solvents in the following volume ratios:
MEK/water: any ratio,
MTBE/acetone/water: 4:4:3,
DCM/MeOH/water: 3:4:3,
where the individual volume ratios deviate from the above values by a maximum of 20%.

2. Method according to claim 1, **characterised by** that the individual volume ratios deviate from the above values by a maximum of 15%.

3. Method according to claim 1, **characterised by** that the individual volume ratios deviate from the above values by a maximum of 10%.

4. Method according to claim 1, **characterised by** that the individual volume ratios deviate from the above values by a maximum of 5%.

5. Method according to any of claims 1 to 4, **characterised by** that the solution to be purified containing 10-deacetylbaccatin III is purified in at least two CPC procedurial steps, and where during the individual CPC procedurial steps one of the following is used as the solvent mixture in such a way that during the method the at least two CPC procedurial steps are carried out with different solvent mixtures: MEK/water, MTBE/acetone/water, and DCM/MeOH/water.

6. Method according to any of claims 1 to 5, **characterised by** that the solution containing 10-deacetylbaccatin III is purified in at least three CPC procedurial steps in such a way that during the method the at least three CPC procedurial steps are performed using different solvent mixtures.

7. Method according to claim 5 or 6, **characterised by** that the CPC procedurial steps are performed one after the other without the interposition of other purification steps.

8. Method according to any of claims 5 to 7, **characterised by** that the CPC procedurial step using the DCM/MeOH/water solvent mixture is performed not as the last CPC procedurial step.

9. Method according to any of claims 1 to 8, **characterised by** that the MEK/water solvent mixture contain the solvents in the 1:2 volume ratio;
where the volume ratio deviates from the above value by a maximum of 20%, preferably by a maximum of 15%, more preferably by a maximum of 10% and most preferably by a maximum of 5%.

10. Method according to any of claims 1 to 9, **characterised by** that pre-purification is implemented for removing the chlorophyll content.

11. Method according to claim 10, **characterised by** that the pre-purification is performed using a CPC procedurial step or a liquid chromatography procedurial step.

12. Method according to any of claims 1 to 11, **characterised by** that the solution to be purified containing 10-deacetylbaccatin III is a plant extract, preferably an extract prepared from any part of a yew tree species, even more preferably an extract made from mainly the leaves of a yew tree species.

13. Method according to claim 12, **characterised by** that the yew tree species is selected from the Taxus Baccata or Taxus Wallichiana species.

## Patentansprüche

1. Zentrifugales Verteilungschromatografie (CPC)-Verfahren, das zur Trennung von 10-Deacetylbaccatin III dient, **dadurch gekennzeichnet, dass** eine zu reinigende Lösung, die 10-Deacetylbaccatin III enthält, in mindestens einem CPC-Verfahrensschritt gereinigt wird, und wobei während des CPC-Verfahrensschritts eines der folgenden als das Lösemittelgemisch verwendet wird: MEK/Wasser, MTBE/Aceton/Wasser und DCM/MeOH/Wasser, wobei die individuellen Lösemittelgemische die Lösemittel in den folgenden Volumenverhältnissen enthalten:
MEK/Wasser: jedes Verhältnis,
MTBE/Aceton/Wasser: 4 : 4: 3,
DCM/MeOH/Wasser: 3 : 4 : 3,
wobei die individuellen Volumenverhältnisse um maximal 20 % von den obigen Werten abweichen.

2. Verfahren nach Anspruch 1, **dadurch gekennzeichnet, dass** die individuellen Volumenverhältnisse um maximal 15 % von den obigen Werten abweichen.

3. Verfahren nach Anspruch 1, **dadurch gekennzeichnet, dass** die individuellen Volumenverhältnisse um maximal 10 % von den obigen Werten abweichen.

4. Verfahren nach Anspruch 1, **dadurch gekennzeichnet, dass** die individuellen Volumenverhältnisse um maximal 5 % von den obigen Werten abweichen.

5. Verfahren nach einem der Ansprüche 1 bis 4, **dadurch gekennzeichnet, dass** die zu reinigende Lösung, die 10-Deacetylbaccatin III enthält, in mindestens zwei CPC-Verfahrensschritten gereinigt wird, und wobei während der individuellen CPC-Verfahrensschritte eines der folgenden in einer Weise als das Lösemittelgemisch verwendet wird, dass während des Verfahrens die mindestens zwei CPC-Verfahrensschritte mit unterschiedlichen Lösemittelgemischen ausgeführt werden: MEK/Wasser, MTBE/Aceton/Wasser und DCM/MeOH/Wasser.

6. Verfahren nach einem der Ansprüche 1 bis 5, **dadurch gekennzeichnet, dass** die zu reinigende Lösung, die 10-Deacetylbaccatin III enthält, in mindestens drei CPC-Verfahrensschritten in einer Weise gereinigt wird, dass während des Verfahrens die mindestens drei CPC-Verfahrensschritte unter Verwendung unterschiedlicher Lösemittelgemische durchgeführt werden.

7. Verfahren nach Anspruch 5 oder 6, **dadurch gekennzeichnet, dass** die CPC-Verfahrensschritte nacheinander ohne das Dazwischenschalten anderer Reinigungsschritte durchgeführt werden.

8. Verfahren nach einem der Ansprüche 5 bis 7, **dadurch gekennzeichnet, dass** der CPC-Verfahrensschritt unter Verwendung des DCM/MeOH/Wasser-Lösemittelgemisches nicht als der letzte CPC-Verfahrensschritt durchgeführt wird.

9. Verfahren nach einem der Ansprüche 1 bis 8, **dadurch gekennzeichnet, dass** das MEK-Wasser-Lösemittelgemisch die Lösemittel im Volumenverhältnis 1: 2 enthält;
wobei das Volumenverhältnis um maximal 20 %, vorzugsweise um maximal 15 %, noch bevorzugter um maximal 10 % und am bevorzugtesten um maximal 5 %, von dem obigen Wert abweicht.

10. Verfahren nach einem der Ansprüche 1 bis 9, **dadurch gekennzeichnet, dass** zum Entfernen des Chlorophyllanteils Vorreinigung vorgenommen wird.

11. Verfahren nach Anspruch 10, **dadurch gekennzeichnet, dass** die Vorreinigung unter Verwendung eines CPC-Verfahrensschritts oder eines Flüssigkeitschromatografie-Verfahrensschritts durchgeführt wird.

12. Verfahren nach einem der Ansprüche 1 bis 11, **dadurch gekennzeichnet, dass** die zu reinigende Lösung, die 10-Deacetylbaccatin III enthält, ein Pflanzenextrakt, vorzugsweise ein aus einem beliebigen Teil einer Eibenbaumspezies präparierter Extrakt, noch bevorzugter ein hauptsächlich aus den Blättern einer Eibenbaumspezies hergestellter Extrakt, ist.

13. Verfahren nach Anspruch 12, **dadurch gekennzeichnet, dass** die Eibenbaumspezies aus der Spezies Taxus Baccata oder Taxus Wallichiana ausgewählt ist.

## Revendications

1. Procédé de chromatographie de partage centrifuge (CPC) servant à séparer la 10-désacétylbaccatine III, **caractérisé en ce qu'**une solution à purifier contenant de la 10-désacétylbaccatine III est purifiée dans au moins une étape procédurale de CPC, et dans lequel pendant l'étape procédurale de CPC l'un des suivants est utilisé comme mélange de solvants : MEK/eau, MTBE/acétone/eau, et DCM/MeOH/eau, dans lequel les mélanges de solvants individuels contiennent les solvants dans les rapports volumiques suivants :
MEK/eau : n'importe quel rapport,
MTBE/acétone/eau : 4:4:3,
DCM/MeOH/eau : 3:4:3,
dans lequel les rapports volumiques individuels s'écartent des valeurs ci-dessus d'un maximum de 20 %.

2. Procédé selon la revendication 1, **caractérisé en ce que** les rapports volumiques individuels s'écartent des valeurs ci-dessus d'un maximum de 15 %.

3. Procédé selon la revendication 1, **caractérisé en ce que** les rapports volumiques individuels s'écartent des valeurs ci-dessus d'un maximum de 10 %.

4. Procédé selon la revendication 1, **caractérisé en ce que** les rapports volumiques individuels s'écartent des valeurs ci-dessus d'un maximum de 5 %.

5. Procédé selon l'une quelconque des revendications 1 à 4, **caractérisé en ce que** la solution à purifier contenant de la 10-désacétylbaccatine III est purifiée dans au moins deux étapes procédurales de CPC, et dans lequel pendant les étapes procédurales individuelles de CPC l'un des suivants est utilisé comme mélange de solvants d'une manière telle que pendant le procédé les au moins deux étapes procédurales de CPC sont effectuées avec différents mélanges de solvants : MEK/eau, MTBE/acétone/eau, et DCM/MeOH/eau.

6. Procédé selon l'une quelconque des revendications 1 à 5, **caractérisé en ce que** la solution contenant de la 10-désacétylbaccatine III est purifiée dans au moins trois étapes procédurales de CPC d'une manière telle que pendant le procédé les au moins trois étapes procédurales de CPC sont effectuées en utilisant différents mélanges de solvants.

7. Procédé selon l'une quelconque des revendications 5 ou 6, **caractérisé en ce que** les étapes procédurales de CPC sont effectuées les unes après les autres sans l'interposition d'autres étapes de purification.

8. Procédé selon l'une quelconque des revendications 5 à 7, **caractérisé en ce que** l'étape procédurale de CPC utilisant le mélange de solvants DCM/MeOH/eau n'est pas effectuée en tant que dernière étape procédurale de CPC.

9. Procédé selon l'une quelconque des revendications 1 à 8, **caractérisé en ce que** le mélange de solvants MEK/eau contient les solvants dans le rapport volumique 1:2 ;
dans lequel le rapport volumique s'écarte de la valeur ci-dessus d'un maximum de 20 %, de préférence d'un maximum de 15 %, plus préférentiellement d'un maximum de 10 % et le plus préférentiellement d'un maximum de 5 %.

10. Procédé selon l'une quelconque des revendications 1 à 9, **caractérisé en ce qu'**une pré-purification est mise en œuvre pour éliminer la teneur en chlorophylle.

11. Procédé selon la revendication 10, **caractérisé en ce que** la pré-purification est effectuée en utilisant une étape procédurale de CPC ou une étape procédurale de chromatographie en phase liquide.

12. Procédé selon l'une quelconque des revendications 1 à 11, **caractérisé en ce que** la solution à purifier contenant de la 10-désacétylbaccatine III est un extrait végétal, de préférence un extrait préparé à partir de n'importe quelle partie d'une espèce d'if, plus préférentiellement un extrait préparé principalement à partir des feuilles d'une espèce d'if.

13. Procédé selon la revendication 12, **caractérisé en ce que** l'espèce d'if est sélectionnée parmi les espèces Taxus Baccata ou Taxus Wallichiana.
